(19) Europäisches Patentamt
European Patent Office
Office européen des brevets

(11) Veröffentlichungsnummer: 0 313 782
A2

(12) EUROPÄISCHE PATENTANMELDUNG

(21) Anmeldenummer: 88114931.4

(22) Anmeldetag: 13.09.88

(51) Int. Cl.4: A61K 31/41 , A61K 31/415 , C07D 249/08 , C07D 233/60

(30) Priorität: 25.09.87 DE 3732388

(43) Veröffentlichungstag der Anmeldung: 03.05.89 Patentblatt 89/18

(84) Benannte Vertragsstaaten: AT BE CH DE ES FR GB GR IT LI NL SE

(71) Anmelder: BAYER AG
Konzernverwaltung RP Patentabteilung
D-5090 Leverkusen 1 Bayerwerk(DE)

(72) Erfinder: Stroech, Klaus, Dr.
Rolsberger Strasse 22
D-5650 Solingen 19(DE)
Erfinder: Bielefeldt, Dietmar, Dr.
Beuthener Strasse 13
D-4030 Ratingen 6(DE)
Erfinder: Plempel, Manfred, Dr.
Zwengenberger Strasse 3c
D-5657 Haan(DE)

(54) **Antimykotische Mittel.**

(57) Die vorliegende Erfindung betrifft neue Azolylmethylcyclopropyl-carbinol-Derivate zur Behandlung von Krankheiten, insbesondere Mykosen.

EP 0 313 782 A2

## Antimykotische Mittel

Die vorliegende Erfindung betrifft neue Azolylmethyl-cyclopropyl-carbinol-Derivate sowie deren pharmakologisch verträgliche Säureadditions-Salze zur Behandlung von Krankheiten, insbesondere Mykosen.

Es ist bereits allgemein bekannt geworden, daß bestimmte Azolylmethyl-cyclopropyl-carbinol-Derivate, wie beispielsweise 1-(4-Chlorphenyl)-1-(1-fluor-cycloprop-1-yl)-2-(1,2,4-triazol-1-yl)-ethan-1-ol und 1-(1-Fluor-cycloprop-1-yl)-1-phenyl-2-(1,2,4-triazol-1-yl)-ethan-1-ol, antimykotische Eigenschaften aufweisen (vergleiche EP-OS 0 180 850). Die Wirkung dieser Stoffe ist jedoch nicht immer in allen Indikationsbereichen voll zufriedenstellend.

Es wurde gefunden, daß die neuen Azolylmethyl-cyclopropyl-carbinol-Derivate der Formel

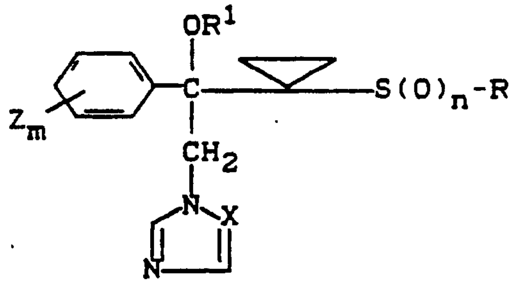

(I)

in welcher

R für Perfluoralkyl mit 1 bis 4 Kohlenstoffatomen, Trichlormethyl, Difluorchlormethyl oder Fluordichlormethyl steht,

$R^1$ für Wasserstoff, Alkyl oder Acyl steht,

Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1.oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht,

m für die Zahlen 0, 1, 2 oder 3 steht und

n für die Zahlen 0, 1 oder 2 steht und

X für Stickstoff oder eine CH-Gruppe steht,

sowie deren Säureadditions-Salze gute antimikrobielle, insbesondere antimykotische Eigenschaften aufweisen.

Die erfindungsgemäß zu verwendenden Azolylmethyl-cyclopropyl-carbinol-Derivate sind durch die Formel (I) allgemein definiert. In dieser Formel stehen vorzugsweise

R für Trifluormethyl, Trichlormethyl, Difluorchlormethyl oder Fluordichlormethyl,

$R^1$ für Wasserstoff, Methyl, Ethyl, n-Propyl, Isopropyl, n-Butyl, Isobutyl, Methylcarbonyl, Ethylcarbonyl, n-Propylcarbonyl, Isopropyl-carbonyl, n-Butylcarbonyl und Isobutyl-carbonyl,

Z für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy,

m für die Zahlen 0, 1, 2 oder 3,

n für die Zahlen 0, 1 oder 2 und

X für Stickstoff oder eine CH-Gruppe.

Wenn m für die Zahlen 2 oder 3 steht, können die für Z stehenden Reste gleich oder verschieden sein.

Bevorzugte erfindungsgemäße Verbindungen sind auch Additionsprodukte aus Säuren und denjenigen Azolylmethyl-cyclopropyl-carbinol-Derivaten der Formel (I), in denen R, $R^1$, X, Z, m und n die Bedeutungen haben, die bereits vorzugsweise für diese Reste und Indices genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Als Beispiele für Azolylmethyl-cyclopropyl-carbinol-Derivate der Formel (I) seien die in der folgenden Tabelle aufgeführten Stoffe genannt.

Tabelle

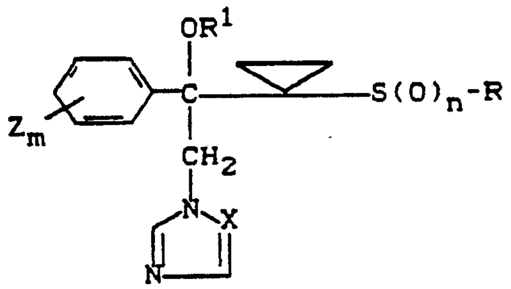

(I)

| $Z_m$ | R | $R^1$ | X | n |
|---|---|---|---|---|
| 4-Cl | $CF_3$ | H | N | 0 |
| 2,4-$Cl_2$ | $CF_3$ | H | N | 0 |
| 2,4-$F_2$ | $CF_3$ | H | N | 0 |
| 4-Cl | $CF_3$ | H | CH | 0 |
| 4-Cl | $CF_3$ | H | CH | 1 |
| 4-Cl | $CF_3$ | H | CH | 2 |
| 4-Cl | $CF_3$ | $CH_3$ | N | 0 |
| 4-$CH_3$ | $CF_3$ | H | N | 0 |
| 4-$CF_3$ | $CF_3$ | H | N | 0 |
| 4-$OCF_3$ | $CF_3$ | H | N | 0 |
| 4-$SCF_3$ | $CF_3$ | H | N | 0 |
| 4-$OCH_3$ | $CF_3$ | H | N | 0 |
| 4-$SCH_3$ | $CF_3$ | H | N | 0 |
| 2,4,6-$Cl_3$ | $CF_3$ | H | N | 0 |

Tabelle (Fortsetzung)

| $Z_m$ | R | $R^1$ | X | n |
|---|---|---|---|---|
| 4- 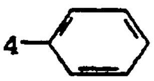 | $CF_3$ | H | N | 0 |
| 4-O- 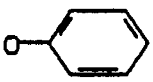 | $CF_3$ | H | N | 0 |
| $4-C_4H_9$-t. | $CF_3$ | H | N | 0 |
| $2-Cl,4-CH_3$ | $CF_3$ | H | N | 0 |
| $2,4-Cl_2$ | $CF_3$ | H | N | 1 |
| $2,4-Cl_2$ | $CF_3$ | H | N | 2 |
| $2,4-Cl_2$ | $CF_3$ | $-CO-CH_3$ | N | 0 |
| $2,4-Cl_2$ | $CF_3$ | $-C_2H_5$ | N | 0 |
| $2,4-Cl_2$ | $CF_3$ | $-CH_3$ | N | 0 |
| $2,4-Cl_2$ | $CF_3$ | H | CH | 0 |
| - | $CF_3$ | H | N | 0 |
| - | $CF_3$ | H | CH | 0 |
| - | $CF_3$ | H | N | 1 |
| - | $CF_3$ | H | N | 2 |
| $2,4-F_2$ | $CF_3$ | H | CH | 0 |
| 4-F | $CF_3$ | H | CH | 0 |
| 4-F | $CF_3$ | $CH_3$ | N | 0 |
| 4-F | $CF_3$ | $CH_3$ | N | 2 |

Die erfindungsgemäß zu verwendenden Azolylmethyl-cyclopropyl-carbinol-Derivate der Formel (I) sowie ihre Säureadditions-Salze sind Gegenstand einer eigenen, älteren Patentanmeldung, die nicht vorveröffentlicht ist (vergleiche die Deutsche Patentanmeldung P 37 20 756 vom 24.06.87). Sie können nach den dort beschriebenen Verfahren erhalten werden, indem man

a) in einer ersten Stufe Phenyl-cyclopropyl-ketone der Formel

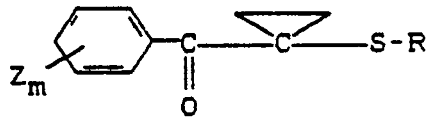 (II)

in welcher
R, Z und m die oben angegebene Bedeutung haben,
entweder
α) mit Dimethyloxosulfonium-methylid der Formel

$$\overset{\delta^{\oplus}\,\delta^{\ominus}}{(CH_3)_2SOCH_2} \qquad (III)$$

oder
β) mit Dimethylsulfonium-methylid der Formel

$$\overset{\delta^{\oplus}\,\delta^{\ominus}}{(CH_3)_2S\ CH_2} \qquad (IV)$$

in Gegenwart von Verdünnungsmitteln, wie beispielsweise Dimethylsulfoxid, bei Temperaturen zwischen 10° C und 60° C umsetzt und
in einer zweiten Stufe die dabei erhaltenen Oxirane der Formel

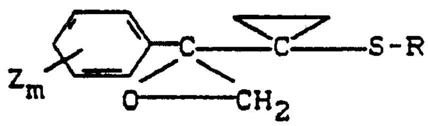 (V)

in welcher
R, Z und m die oben angegebene Bedeutung haben,
mit Azolen der Formel

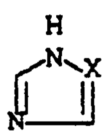 (VI)

in welcher
X die oben angegebene Bedeutung hat,
in Gegenwart von Säurebindemitteln, wie beispielsweise Alkalialkoholaten und in Gegenwart von Verdünnungsmitteln, wie beispielsweise Dimethylformamid, bei Temperaturen zwischen 60° C und 150° C umsetzt,
oder
b) Azolylmethyl-cyclopropyl-carbinol-Derivate der Formel

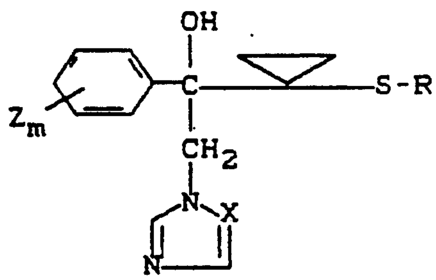

(Ia)

in welcher
R, X, Z und m die oben angegebene Bedeutung haben,
mit Oxidationsmitteln gegebenenfalls in Gegenwart von Verdünnungsmitteln, wie Wasserstoffperoxid/Eisessig, bei Temperaturen zwischen -30° C und +90° C umsetzt,
oder

c) Azolylmethyl-cyclopropyl-carbinol-Derivate der Formel

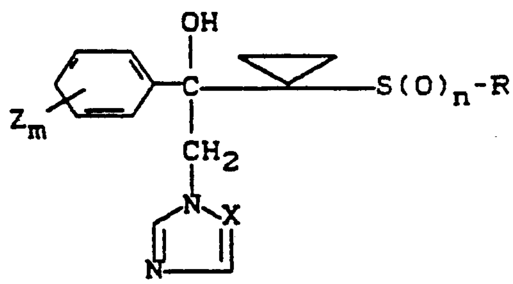

(Ib)

in welcher
R, Z, X, m und n die oben angegebene Bedeutung haben,
mit starken Basen, wie beispielsweise Alkalimetallamiden oder -hydriden in Gegenwart von Verdünnungsmitteln, wie beispielsweise Dioxan, bei Temperaturen zwischen 20° C und 100° C umsetzt und die dabei entstehenden Alkoholate der Formel

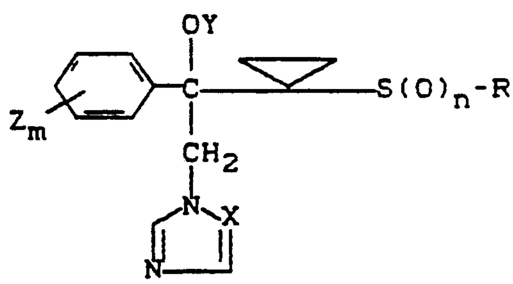

(Ic)

in welcher
R, X, Z, m und n die oben angegebene Bedeutung haben und
Y für einen kationischen Rest einer Base steht,
mit Halogenverbindungen der Formel

$R^2$-Hal (VII)

in welcher
$R^2$ für Alkyl oder Acyl steht und
Hal für Halogen steht,
in Gegenwart eines Verdünnungsmittels, wie beispielsweise Dioxan, bei Temperaturen zwischen 20° C und 100° C umsetzt,
und gegebenenfalls anschließend an die so erhaltenen Verbindungen der Formel (I) in üblicher Weise eine Säure addiert.

Die erfindungsgemäßen Stoffe enthalten ein asymmetrisch substituiertes Kohlenstoffatom. Sie können daher in optischen Isomerenformen anfallen. Die vorliegende Erfindung betrifft sowohl die einzelnen Isomeren als auch deren Gemische.

Die Phenyl-cyclopropyl-ketone der Formel (II) sind bisher noch nicht bekannt, auch sie sind Gegenstand der o.g. eigenen, älteren Deutschen Patentanmeldung. Sie lassen sich herstellen, indem man

d) in einer ersten Stufe Phenylketone der Formel

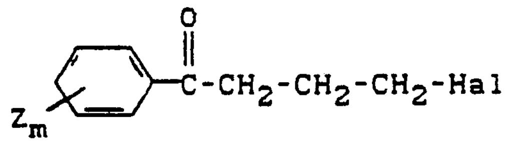

(VIII)

in welcher
Z und m die oben angegebene Bedeutung haben und
Hal für Chlor oder Brom steht,
mit Mercaptanen der Formel

Hal′-S-R (IX)

in welcher
R die oben angegebene Bedeutung hat und
Hal′ für Chlor oder Brom steht,
in Gegenwart eines Verdünnungsmittels umsetzt und die entstehenden Phenylpropyl-ketone der Formel

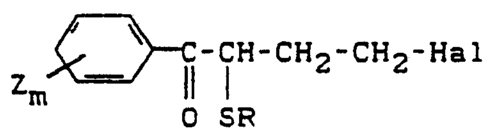

(X)

in welcher
R, Z, Hal und m die oben angegebene Bedeutung haben,
in einer zweiten Stufe in Gegenwart eines Säurebindemittels und in Gegenwart eines Verdünnungsmittels umsetzt.

Die bei dem Verfahren (d) als Ausgangsstoffe benötigten Phenylketone der Formel (VIII) sind bekannt oder lassen sich nach prinzipiell bekannten Verfahren in einfacher Weise herstellen (vgl. DE-OS 2 521 104, DE-OS 2 320 355 und DE-OS 2 351 948). So erhält man Phenylketone der Formel (VIII), indem man Benzol-Derivate der Formel

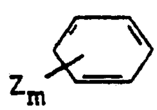

(XI)

in welcher
Z und m die oben angegebene Bedeutung haben,
mit Buttersäurehalogeniden der Formel

$$Cl-\overset{O}{\overset{\|}{C}}-CH_2-CH_2-CH_2-Hal \quad (XII)$$

in welcher
Hal die oben angegebene Bedeutung hat,
in Gegenwart eines Friedel-Crafts-Katalysators, wie zum Beispiel Aluminiumchlorid, gegebenenfalls in Gegenwart eines Verdünnungsmittels, wie zum Beispiel Methylenchlorid, bei Temperaturen zwischen -15°

C und +60° C umsetzt.

Die bei dem Verfahren (d) weiterhin als Ausgangsstoffe benötigten Mercaptane der Formel (IX) sind bekannt.

Als Verdünnungsmittel kommen bei der Durchführung der ersten Stufe des Verfahrens (d) alle für derartige Umsetzungen üblichen inerten organischen Solventien in Betracht. Vorzugsweise verwendbar sind halogenierte aliphatische Kohlenwasserstoffe, wie zum Beispiel Methylenchlorid, Chloroform und Tetrachlorkohlenstoff.

Die Reaktionstemperaturen können bei der Durchführung der ersten Stufe des Verfahrens (d) in einem bestimmten Bereich variiert werden. Im allgemeinen arbeitet man bei Temperaturen zwischen 0° C und 80° C, vorzugsweise zwischen 10 und 50° C.

Bei der Durchführung der ersten Stufe des Verfahrens (d) setzt man auf 1 Mol an Phenylketon der Formel (VIII) im allgemeinen 2 bis 4 Mol an Mercaptanen der Formel (IX) ein. Die Isolierung der entstehenden Phenyl-propylketone der Formel (X) erfolgt nach üblichen Methoden. Im allgemeinen geht man so vor, daß man das Lösungsmittel abzieht und das verbleibende Produkt entweder direkt oder nach vorheriger Reinigung für die weitere Synthese einsetzt.

Bei der Durchführung der zweiten Stufe des Verfahrens (d) kommen als Säurebindemittel alle für derartige Umsetzungen üblichen Basen in Betracht. Vorzugsweise verwendbar sind Alkoholate, wie Natriummethylat oder Kalium-tert.-butylat, ferner niedere tertiäre Alkylamine, Cycloalkylamine und Aryl-alkyl-amine, wie Triethylamin, Dimethyl-anilin, 1,5-Diaza-bicyclo[4.3.0]non-5-en und 1,8-Diazabicyclo[5.4.0]undecen.

Als Verdünnungsmittel kommen bei der Durchführung der zweiten Stufe des Verfahrens (d) alle für derartige Umsetzungen üblichen inerten organischen Solventien in Frage. Vorzugsweise verwendbar sind Alkohole, wie Methanol, Ethanol, Methoxyethanol, Propanol oder tert.-Butanol, ferner Ketone, wie Aceton und Butan-2-on, außerdem Nitrile, wie Acetonitril, weiterhin Ester, wie Essigester, darüber hinaus Ether, wie Dioxan, aromatische Kohlenwasserstoffe, wie Benzol oder Toluol, und auch Amide, wie Dimethylformamid.

Die Temperaturen können auch bei der Durchführung der zweiten Stufe des Verfahrens (d) in einem größeren Bereich variiert werden. Im allgemeinen arbeitet man bei Temperaturen zwischen 0° C und 100° C, vorzugsweise zwischen 20° C und 60° C.

Bei der Durchführung der zweiten Stufe des Verfahrens (d) setzt man auf 1 Mol an Phenyl-propyl-keton der Formel (X) im allgemeinen 1 bis 3 Mol an Base ein. Die Aufarbeitung erfolgt nach üblichen Methoden. Im allgemeinen geht man so vor, daß man das Reaktionsgemisch mit Wasser vermengt, anschließend mit einem in Wasser wenig löslichen organischen Lösungsmittel extrahiert, die vereinigten organischen Phasen mit Wasser wäscht und nach dem Trocknen einengt. Das verbleibende Produkt kann nach üblichen Methoden, wie zum Beispiel Säulenchromatographie, von eventuell noch vorhandenen Verunreinigungen befreit werden.

Das bei dem erfindungsgemäßen Verfahren (a) als Reaktionskomponente benötigte Dimethyl-oxosulfonium-methylid der Formel (III) ist bekannt (vgl. J. Am. Chem. Soc. 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid oder Natriumamid, insbesondere mit Kalium-tert.-butylat oder Natriummethylat, in Gegenwart eines Verdünnungsmittels erzeugt.

Das bei dem erfindungsgemäßen Verfahren (a) außerdem als Reaktionskomponente in Betracht kommende Dimethylsulfonium-methylid der Formel (IV) ist ebenfalls bekannt (vgl. Heterocycles 8, 397 (1977)). Es wird bei der obigen Umsetzung ebenfalls in frisch hergestelltem Zustand eingesetzt, indem man es in situ zum Beispiel aus Trimethylsulfonium-halogenid oder Trimethylsulfonium-methylsulfat, in Gegenwart einer starken Base, wie zum Beispiel Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Die bei dem erfindungsgemäßen Verfahren (a) als Zwischenprodukte auftretenden Oxirane der Formel (V) sind noch nicht bekannt. Sie stellen allgemein interessante Zwischenprodukte dar.

Die für die zweite Stufe des erfindungsgemäßen Verfahrens (a) als Reaktionskomponenten benötigten Azole der Formel (VI) sind allgemein bekannte Verbindungen der organischen Chemie.

Die für das erfindungsgemäße Verfahren (b) als Ausgangsstoffe benötigten Azolylmethyl-cyclopropyl-carbinol-Derivate der Formel (Ia) sind erfindungsgemäße Verbindungen.

Als Reaktionskomponenten kommen bei dem erfindungsgemäßen Verfahren (b) alle für derartige Umsetzungen übliche Oxidationsmittel in Betracht. Vorzugsweise verwendbar sind Wasserstoffperoxid und Persäuren, wie m-Chlorperbenzoesäure und Peressigsäure.

Bei der Durchführung des erfindungsgemäßen Verfahrens (b) setzt man auf 1 Mol der erfindungsgemäßen Verbindungen der Formel (Ia) etwa 1 bis 5 Mol Oxidationsmittel ein. Bei der Anwendung von 1 Mol Oxidationsmittel, wie m-Chlorperbenzoesäure in Methylenchlorid oder Wasserstoffperoxid in Essigsäure

oder Acetanhydrid bei Temperaturen zwischen -30° C bis +90° C, entstehen vorzugsweise die erfindungsgemäßen Verbindungen der Formel (I) mit der -SO-Gruppierung. Bei Überschuß an Oxidations mittel und Temperaturen zwischen 10° C und 90° C entstehen vorzugsweise die erfindungsgemäßen Verbindungen der Formel (I) mit einer -$SO_2$-Gruppierung. Die Isolierung der Oxidationsprodukte erfolgt in üblicher Weise.

Die für das erfindungsgemäße Verfahren (c) als Ausgangsstoffe benötigten Azolylmethyl-cyclopropyl-carbinol-Derivate der Formel (Ib) sind ebenfalls erfindungsgemäße Verbindungen. Ihre Überführung in die entsprechenden Alkoholate erfolgt in allgemein bekannter Weise, indem man mit geeigneten starken Basen, wie Alkalimetall-amiden oder -hydriden, quarternären Ammonium-hydroxiden oder Phosphonium-hydroxiden in einem inerten Verdünnungsmittel, wie zum Beispiel Dioxan, bei Raumtemperatur umsetzt. Demgemäß steht Y in den Verbindungen der Formel (Ic) vorzugsweise für ein Alkalimetallkation, wie ein Natrium- oder Kaliumkation, oder für ein quarternäres Ammonium- oder Phosphoniumkation.

Die außerdem für das erfindungsgemäße Verfahren (c) als Ausgangsstoffe zu verwendenden Halogenverbindungen der Formel (VII) sind bekannt oder lassen sich nach im Prinzip bekannten Methoden herstellen.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäße Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:

Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einen Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder oder Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure re, (b) Bindemittel, z.B. Carboxymethylcellulose, Alginate, Gelatine, Polyvinylpyrrolidon, (c) Feuchthaltemittel, z.B. Glycerin, (d) Sprengmittel z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Den Tabletten, Dragees, Kapseln, Pillen und Granulaten können mit den üblichen gegebenenfalls

Opakisierungsmittel enthaltenen Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Träger stoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silocone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungs gemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise 5 bis 150 mg/kg Körpergewicht je 24 Stunden, gegebenenfalls in Form mehrerer Einzelgaben zur Erzielung der gewünschten Ergebnisse zu verabreichen.

Bei oralen Applikationen werden die erfindungsgemäßen Wirkstoffe in Gesamtmengen von etwa 2,5 bis etwa 200, vorzugsweise 5 bis 150 mg/kg Körpergewicht je 24 Stunden und bei parenteraler Applikation in Gesamtmengen von etwa 2,5 bis etwa 50, vorzugsweise 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objektes, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens

leicht erfolgen.

Herstellungsbeispiele

**Beispiel 1**

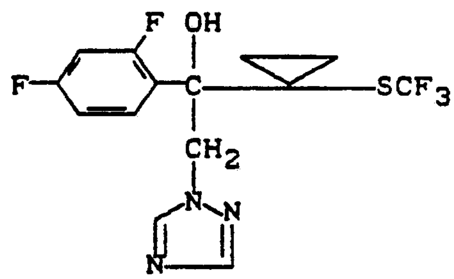

(I-1)

In ein Gemisch aus 12 g (174 mMol) 1,2,4-Triazol und 1,4 g (12,5 mMol) Kalium-tert.-butylat in 30 ml absolutem Dimethylformamid wird unter Stickstoffatmosphäre und unter Rühren eine Lösung von 15,9 g (54 mMol) 2-(2,4-Difluorphenyl)-2-[1-(trifluormethylmercapto)-cycloprop-1-yl]-oxiran in 20 ml absolutem Dimethylformamid eingetropft. Das Reaktionsgemisch wird 8 Stunden bei 100° C gerührt. Danach zieht man das Lösungsmittel unter vermindertem Druck ab, nimmt den Rückstand in Essigsäureethylester auf, wäscht zweimal mit Wasser und engt nach dem Trocknen über Natriumsulfat unter vermindertem Druck ein. Der Rückstand wird mit Chloroform als Laufmittel über eine Kieselgelsäule chromatographiert. Man erhält auf diese Weise 12,5 g (65 % der Theorie) an 1-(2,4-Difluorphenyl)-1-hydroxy-2-(1,2,4-triazol-1-yl)-1-(1-trifluormethylmercapto-cycloprop-1-yl)-ethan vom Schmelzpunkt 103° C.

Herstellung von Ausgangsprodukten

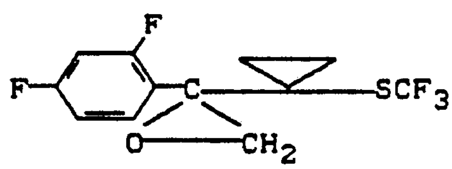

(V-1)

In ein Gemisch von 1,9 g (63 mMol) Natriumhydrid (80 %ig) und 13,5 g (61 mMol) Trimethyloxosulfoniumiodid werden bei 10° C unter Stickstoffatmosphäre 50 ml absolutes Dimethylsulfoxid getropft. Das Gemisch wird noch eine Stunde bei 10° C gerührt und dann bei der gleichen Temperatur tropfenweise mit einer Lösung von 15,3 g (54 mMol) 2,4-Difluorphenyl-(1-trifluormethylmercapto-cycloprop-1-yl)-keton in 20 ml abolutem Dimethylsulfoxid versetzt. Man rührt 48 Stunden bei Raumtemperatur und anschließend 1 Stunde bei 40° C. Das Reaktionsgemisch wird auf Wasser gegossen. Man extrahiert mit Essigsäureethylester, wäscht die vereinigten organischen Phasen mit Wasser, trocknet über Natriumsulfat und engt unter vermindertem Druck ein. Man erhält 15,9 g (99 % der Theorie) an 2-(2,4-Difluorphenyl)-2-[1-(trifluormethylmercapto)-cycloprop-1-yl]-oxiran in Form eines farblosen Öles.

$^1$H-NMR (360 MHz; $CDCl_3$):

δ =

1,00-1,50 (m, 4H); 3,04 (m,2H);

6,80-6,96 (m, 2H); 7,38-7,48 (m,1H).

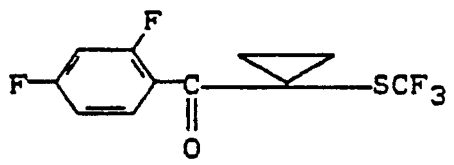

(II-1)

Eine Lösung von 47 g (0,15 Mol) (3-Chlor-1-trifluormethylmercapto-propyl)-(2,4-difluorphenyl)-keton in 70 ml tert.-Butanol wird bei 40° C unter Rühren in ein Gemisch aus 20 g (0,18 Mol) Kalium-tert.-butylat und 50 ml tert.-Butanol getropft. Das Reaktionsgemisch wird 4 Stunden bei 40° C gerührt und dann auf Wasser gegossen. Man extrahiert mit Essigsäureethylester, wäscht die vereinigten organischen Phasen mit Wasser, trocknet über Natriumsulfat und engt durch Abziehen des Lösungsmittels unter vermindertem Druck ein. Der verbleibende Rückstand wird über eine Kieselgelsäule mit Petrolether: Methylenchlorid = 1:0,4 als Laufmittel chromatographiert. Man erhält 15,3 g (36 % der Theorie) 2,4-Difluorphenyl-(1-trifluormethylmercapto-cycloprop-1-yl)-keton in Form eines farblosen Öles.

$^{1}$H-NMR (200 MHz, $CDCl_3$):
δ =
1,57 (m, 2H); 1,95 (m, 2H);
6,80-7,02 (m, 2H); 7,55-7,68 (m, 1H).

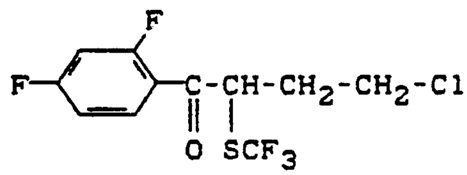

(X-1)

In eine Lösung von 43 g (0,2 Mol) 3-Chlorpropyl-2,4-difluorphenyl-keton in 100 ml Methylenchlorid werden bei 20 bis 25° C unter Rühren 75 g (0,55 Mol) Trifluormethylsulfenylchlorid gegeben. Das Gemisch wird 108 Stunden bei Raumtemperatur gerührt und dann durch Abziehen des Lösungsmittels unter vermindertem Druck eingeengt. Man erhält 63 g (3-Chlor-1-trifluormethylmercapto-propyl)-2,4-difluorphenyl-keton.

$^{19}$F-NMR (Externer Standard: $CF_3COOH$):
δ =
- 36,6 ppm (Duplett)
+ 24,5 ppm (Multiplett)
+ 27,9 ppm (Multiplett)

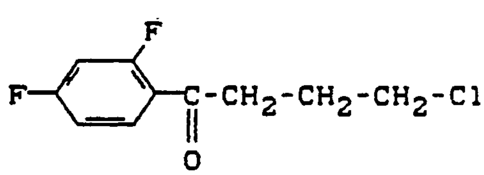

(VIII-1)

In ein Gemisch von 50 g (0,44 Mol) 1,3-Difluorbenzol und 64 g (0,48 Mol) wasserfreiem Aluminiumchlorid werden bei 20° C unter Rühren 62 g (0,44 Mol) 4-Chlorbuttersäurechlorid getropft. Das Reaktionsgemisch wird 3,5-Stunden bei 30° C gerührt bis eine klare Lösung entstanden ist und die Gasentwicklung nachgelassen hat. Nach dem Abkühlen auf Raumtemperatur werden 300 ml Methylenchlorid zugegeben. Man gießt die entstandene Lösung auf 300 g Eis, trennt die organische Phase ab, trocknet über Natriumsulfat und engt durch Abziehen des Lösungsmittels unter vermindertem Druck ein. Man erhält 83 g (86 % der Theorie) an 3-Chlorpropyl-2,4-difluorphenyl-keton in Form einer Flüssigkeit.

Kp = 80° C / 0,1 mbar

Beispiel 2

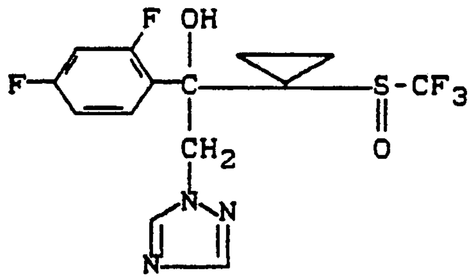

(I-2)

In eine Lösung von 4 g 1-(2,4-Difluorphenyl)-1-hydroxy-2-(1,2,4-triazol-1-yl)-1-(1-trifluormethylmercapto-cycloprop-1-yl)-ethan in 30 ml Eisessig werden bei 85° C unter Rühren 6 ml 35 %ige Wasserstoffperoxid-Lösung eingetropft. Das Reaktionsgemisch wird 4 Stunden bei 85° C gerührt und dann auf 80 ml Wasser gegossen. Durch Zugabe von verdünnter, wäßriger Natronlauge wird die Lösung basisch gestellt. Man extrahiert mehrfach mit Methylenchlorid, wäscht die vereinigten organischen Phasen mit verdünnter, wäßriger Natronlauge und Wasser, trocknet dann über Natriumsulfat und engt durch Abziehen des Lösungsmittels unter vermindertem Druck ein. Der verbleibende Rückstand wird an Kieselgel chromatographisch gereinigt. Man erhält 1,7 g (41 % der Theorie) an 1-(2,4-Difluorphenyl)-1-hydroxy-2-(1,2,4-triazol-1-yl)-1-(trifluormethylsulfoxy-cycloprop-1-yl)-ethan vom Schmelzpunkt 101° C.

Nach der im Beispiel 1 angegebenen Methode wurden auch die in den folgenden Beispielen aufgeführten Verbindungen hergestellt:

**Beispiel 3**

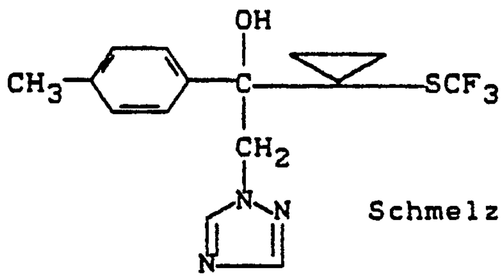

(I-3)

Schmelzpunkt 127° C

**Beispiel 4**

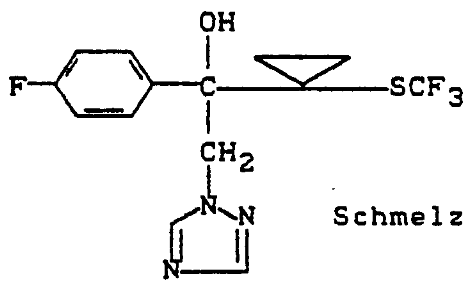

(I-4)

Schmelzpunkt 128° C

**Beispiel 5**

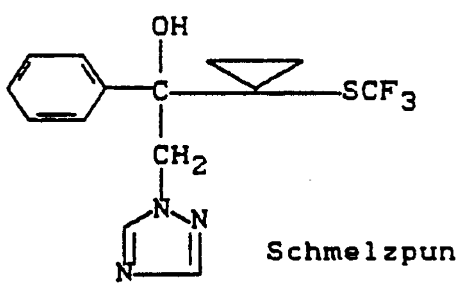

(I-5)

Schmelzpunkt 124° C

Verwendungsbeispiele

In dem folgenden in-vivo-Test werden die nachstehend angegebenen Verbindungen als Vergleichssubstanzen eingesetzt:

(A) 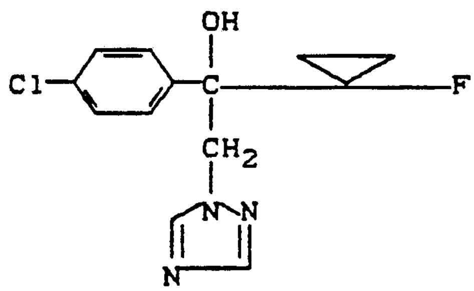

(B) 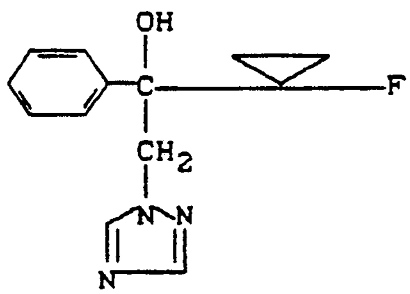

(bekannt aus EP-OS 0 180 850)

Beispiel A

Antimykotische in-vivo-Wirksamkeit (oral) bei Mäusecandidose

Versuchsbeschreibung:

Mäuse vom Typ SPF-$CF_1$ wurden intravenös mit 1 - 2 x $10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 10 - 100 mg/kg Körpergewicht der Präparate oral behandelt.

Ergebnis:

Unbehandelte Tiere starben 3 bis 6 Tage post infektionem. Die Überlebensrate am 6.Tag post infektionem betrug bei unbehandelten Kontrolltieren etwa 5 %.

In diesem Test zeigen z.B. die erfindungsgemäßen Verbindungen (I-1), (I-2) und (I-4) eine bessere Wirkung als die aus dem Stand der Technik bekannten Verbindungen (A) und (B).

Tabelle A

| Antimykotische in-vivo-Wirkung (oral) bei Mäuse-Candidose | |
|---|---|
| Wirkstoff | Wirkung |
| (A)(bekannt)<br>(B)(bekannt) | k.W.<br>k.W. |
| Verbindungen gemäß Herstellungsbeispiel | |
| (I-1)<br>(I-2)<br>(I-4) | + + + + +<br>+ + + + +<br>+ + + + + |
| Zeichenerklärung: | |
| + + + + + = sehr gute Wirkung = 90 % Überlebende am 6.Tag.p.i.<br>+ + + + = gute Wirkung = 80 % Überlebende am 6.Tag.p.i.<br>+ + + = Wirkung = 60 % Überlebende am 6.Tag.p.i.<br>+ + = schwache Wirkung = 40 % Überlebende am 6.Tag.p.i.<br>+ = Spur Wirkung = unter 40 % Überlebende am 6. Tag p.i.<br>k.W. = kein Unterschied zur unbehandelten Infektionskontrolle | |

Beispiel B : Formulierungen

| 1.) Lösung: | | |
|---|---|---|
| Wirkstoff gemäß Formel (I) | : | 10 g |
| Alkohol, rein (96 %ig) | : | 300 g |
| Isopropylmyristat | : | 526 g |
| | | 836 g |

| 2.) Creme: | | |
|---|---|---|
| Wirkstoff gemäß Formel (I) | : | 10 g |
| Aralcel 60 | : | 20 g |
| (Sorbitan-monostearat) | | |
| Tween 60 | : | 15 g |
| (Polyoxyethylen (2) -sorbitan-monostearat) | | |
| Walrat, künstlich | : | 30 g |
| (Mischung vom Estern von gesättigten Fettsäuren $C_{14}$-$C_{18}$ und Fettalkoholen $C_{14}$-$C_{18}$) | | |
| Lanette O | : | 100 g |
| Eutanol G | : | 135 g |
| (2-Octyl-dodecanol) | | |
| Benzylalkohol | : | 10 g |
| Wasser, entmineralisiert | : | 680 g |
| | | 1000 g |

## Ansprüche

1. Azolylmethylcyclopropylcarbinol-Derivate der allgemeinen Formel

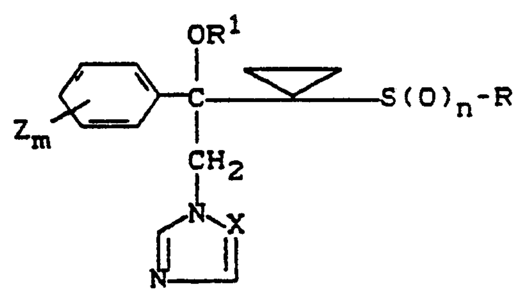

in welcher unabhängig voneinander
R für Perfluoralkyl mit 1 bis 4 Kohlenstoffatomen, Trichlormethyl, Difluorchlormethyl oder Fluordichlormethyl steht,
R' für Wasserstoff, Alkyl oder Acyl steht,
Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht,
m für die Zahlen 0, 1, 2 oder 3 steht und
n für die Zahlen 0, 1 oder 2 steht und
X für Stickstoff oder eine CH-Gruppe steht,
sowie deren Säureadditions-Salze zur Bekämpfung von Krankheiten.

2. Azolylmethylcyclopropylcarbinol-Derivate nach Anspruch 1, in denen unabhängig voneinander
R für Trifluormethyl, Trichlormethyl, Difluorchlormethyl oder Fluordichlormethyl,
R' für Wasserstoff, Methyl, Ethyl, n-Propyl, Isopropyl, n-Butyl, Isobutyl, Methylcarbonyl, Ethylcarbonyl, n-Propylcarbonyl, Isopropylcarbonyl, n-Butylcarbonyl und Isobutylcarbonyl,
Z für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenoxy,
m für die Zahlen 0, 1, 2 oder 3,
n für die Zahlen 0, 1 oder 2 und
X für Stickstoff oder eine CH-Gruppe steht,
sowie deren Säureadditions-Salze zur Bekämpfung von Krankheiten.

3. 1-(2,4-Difluorphenyl)-1-hydroxy-2-(1,2,4-triazol-1-yl)-1-(1-trifluormethylmercaptocycloprop-1-yl)-ethan

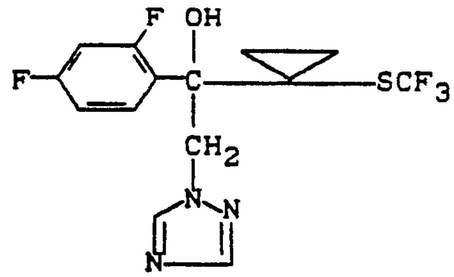

sowie dessen Säureadditions-Salze zur Bekämpfung von Krankheiten.

4. 1-(2,4-Difluorphenyl)-1-hydroxy-2-(1,2,4-triazol-1-yl)-1-(trifluormethylsulfoxycycloprop-1-yl)-ethan

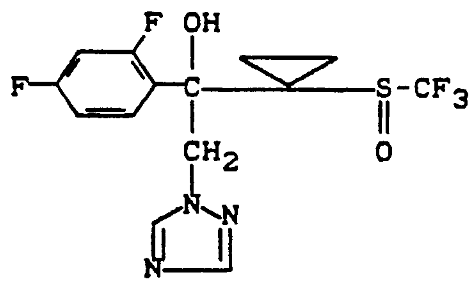

sowie dessen Säureadditions-Salze zur Bekämpfung von Krankheiten.

5. Azolylmethylcyclopropylcarbinol-Derivate nach Ansprüchen 1 - 4 zur Bekämpfung von Mykosen.

6. Arzneimittel, enthaltend Azolylmethylcyclopropylcarbinol-Derivate nach Ansprüchen 1 - 4.

7. Antimykotische Mittel, enthaltend Azolylmethylcyclopropylcarbinol-Derivate nach Ansprüchen 1 - 4.

8. Verwendung der Azolylmethylcyclopropylcarbinol-Derivate nach Ansprüchen 1 - 4 bei der Herstellung von Arzneimitteln zur Bekämpfung von Krankheiten.

9. Verwendung der Azolylmethylcyclopropylcarbinol-Derivate nach Ansprüchen 1 - 4 bei der Herstellung von Arzneimitteln zur Bekämpfung von Mykosen.